(19) Europäisches Patentamt
European Patent Office
Office européen des brevets

(11) EP 0 798 289 B1

(12) **EUROPÄISCHE PATENTSCHRIFT**

(45) Veröffentlichungstag und Bekanntmachung des Hinweises auf die Patenterteilung:
**07.03.2001 Patentblatt 2001/10**

(51) Int Cl.[7]: **C07C 251/12**, C07C 227/32

(21) Anmeldenummer: **97104673.5**

(22) Anmeldetag: **19.03.1997**

(54) **Alpha-Iminocarbonsäurederivate**

Alpha-Iminocarboxylic acid derivative

Dérivés de l'acide alpha-iminocarboxylique

(84) Benannte Vertragsstaaten:
**AT BE CH DE DK ES FR GB IE IT LI NL**

(30) Priorität: **29.03.1996 EP 96105034**

(43) Veröffentlichungstag der Anmeldung:
**01.10.1997 Patentblatt 1997/40**

(73) Patentinhaber: **F. HOFFMANN-LA ROCHE AG**
**4070 Basel (CH)**

(72) Erfinder:
- **Broger, Emil Albin**
  **4312 Magden (CH)**
- **Schmid, Rudolf**
  **4144 Arlesheim (CH)**

(74) Vertreter: **Mezger, Wolfgang, Dr. et al**
**F.Hoffmann-La Roche AG**
**Patent Department (PLP),**
**124 Grenzacherstrasse**
**4070 Basel (CH)**

(56) Entgegenhaltungen:
**FR-A- 2 170 180**

- **BULLETIN OF THE CHEMICAL SOCIETY OF JAPAN, Bd. 61, Nr. 4, April 1988, Seiten 1425-1427, XP000673451 MUNEGUMI T ET AL: "ASYMMETRIC CATALYTIC HYDROGENATIONS OF OXIMES AND BENZYLIMINO DERIVATIVES OF CHIRAL PYRUVAMIDES"**
- **BULLETIN DE LA SOCIETE CHIMIQUE DE FRANCE, Nr. 6, November 1986, Seiten 864-867, XP000673452 REMLI M ET AL: "STUDY OF REACTIVITY OF 3-FLUORO 2-OXO ACID DERIVATIVES SYNTHESIS OF FLUORINATED ALCOHOLS, SCHIFF BASES, OXIMES, HYDRAZONES, AND QUINOXALINES DERIVATIVES"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 798 289 B1

Printed by Jouve, 75001 PARIS (FR)

## Beschreibung

Die Erfindung betrifft optisch aktive α-Iminocarbonsäurederivate der Formel

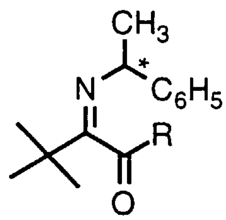
I

worin
R für OH, $NH_2$, nieder-Alkyl-NH oder Phenyl-niederalkyl-NH, insbesondere für

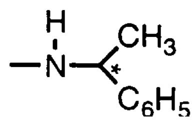
($R^0$)

steht.

Im Rahmen der Erfindung bezeichnet "nieder-Alkyl" geradkettige oder verzweigte Alkylgruppen mit bis zu 6, vorzugsweise bis zu 4 C-Atomen, wie Methyl, Ethyl oder tert-Butyl. Ein Beispiel für durch Phenyl substituiertes nieder-Alkyl ist 1-Phenethyl.

Beispiele von Verbindungen der Formel I sind:

(S)-3,3,N-Trimethyl-2-(1-phenylethylimino)-butyramid,
(R)-3,3,N-Trimethyl-2-(1-phenylethylimino)-butyramid,
(S)-3,3-Dimethyl-N-[(S)-1-phenylethyl]-2-(1-phenylethylimino)-butyramid,
(R)-3,3-Dimethyl-N-[(R)-1-phenylethyl]-2-(1-phenylethylimino)-butyramid,
(S)-3,3-Dimethyl-2-(1-phenylethylimino)-buttersäure,
(R)-3,3-Dimethyl-2-(1-phenylethylimino)-buttersäure,
(S)-N-tert-Butyl-3,3-dimethyl-2-(1-phenylethylimino)-butyramid,
(R)-N-tert-Butyl-3,3-dimethyl-2-(1-phenylethylimino)-butyramid,
(S)-3,3-Dimethyl-2-(1-phenylethylimino)-butyramid und
(R)-3,3-Dimethyl-2-(1-phenylethylimino)-butyramid.

Die Erfindung betrifft ferner ein Verfahren zur Herstellung der enantiomeren α-Aminocarbonsäurederivate der Formel

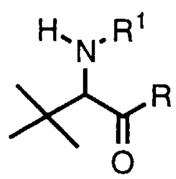
II

worin $R^1$ für H oder eine Gruppe der Formel

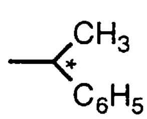
($R^{10}$)

steht und R die obige Bedeutung hat.

Dieses Verfahren ist dadurch gekennzeichnet, dass man eine Verbindung der Formel I katalytisch hydriert, insbesondere unter Verwendung eines Platin- oder vorzugsweise Palladiumkatalysators.

Die Verbindungen der Formel II sind wertvolle Zwischenprodukte in der Synthese von therapeutischen Pseudopeptiden. So können die Verbindungen II, worin $R^1$ eine Gruppe der Formel $R^{10}$ ist (vgl. nachstehende Formel IIa), wie nachstehend beschrieben in die Verbindungen II, worin $R^1$ für H steht (vgl. Formel IIb), übergeführt werden. Letztere Verbindungen sind in den europäischen Patentanmeldungen Nr. 497 192 und Nr. 664 284 als Ausgangsmaterialien zur Herstellung von Kollagenase-Inhibitoren beschrieben.

Optisch reines tert-Leucin, d.h. die Verbindung der Formel II, worin R für OH und $R^1$ für H stehen (vgl. nachstehende Verbindung IIb, worin R OH ist), ist ein Handelsprodukt, das in verschiedenen asymmetrischen Synthesen verwendet werden kann, z.B. bei der Herstellung von optisch reinen unnatürlichen Aminosäuren.

Weitere Beispiele von Verbindungen der Formel II sind:

(S)-tert-Leucinamid,
(S)-N-Methyl-tert-leucinamid,
(R)-N-Methyl-tert-leucinamid,
(S)-N-tert-Butyl-tert-leucinamid,
(S)-3,3,N-Trimethyl-2-[(S)-1-phenyl-ethylamino]-butyramid,
(R)-3,3,N-Trimethyl-2-[(S)-1-phenyl-ethylamino]-butyramid,
(S)-N-[(S)-1-Phenyl-ethyl]-tert-leucinamid.

Die Hydrierung I → II wird in einem Lösungsmittel, wie Methanol, in Gegenwart eines heterogenen Katalysators, der zweckmässigerweise aus einem Edelmetall, wie Pd oder Pt, auf einem Träger, wie Kohle, besteht und mit einer Base, wie Triethylamin, modifiziert sein kann. Zweckmässigerweise wird die Hydrierung bei einer Temperatur bis zu etwa 80°C, unter einem Druck von bis zu etwa 5 bar, bewerkstelligt. Die Hydrierung einer α-Iminocarbonsäure der Formel I, worin R für OH steht, wird zweckmässigerweise mit einem Pd-Katalysator in Methanol in Gegenwart einer Säure, wie Essigsäure oder vorzugsweise Weinsäure, bewerkstelligt. Man erhält dabei optisch reines tert-Leucin.

Die Hydrierung verläuft über die Verbindungen der Formel

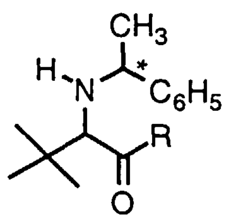

IIa

die man entweder in situ oder nach Isolierung in die Verbindungen der Formel

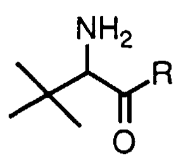

IIb

durch Hydrogenolyse überführen kann. Mit einem Pt-Katalysator oder einem mit Base modifiziertem Pd-Katalysator lässt sich die Verbindung IIa leicht isolieren und zweckmässigerweise mit einem Pd-Katalysator in die Verbindung IIb hydrogenolisieren.

Eine Verbindung der Formel II, worin $R^1$ für H und R für $NH_2$, nieder-Alkyl-NH oder Phenyl-niederalkyl-NH steht, kann man mit einer Säure, wie Salzsäure, zu tert-Leucin hydrolysieren.

Erfindungsgemäss werden die α-Iminocarbonsäurederivate der Formel I dadurch hergestellt, dass man

a) zur Herstellung der α-Iminocarbonsäure der Formel I, worin R für OH steht, die Trimethylbrenztraubensäure

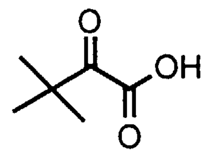 3

mit einer Verbindung der Formel

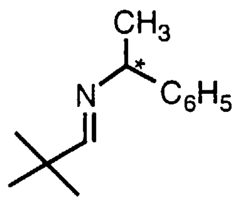 IV

umsetzt,

b) zur Herstellung eines α-Iminocarboxamids der Formel I, worin R für $NH_2$, nieder-Alkyl-NH oder Phenyl-nieder-alkyl-NH, insbesondere für $R^0$, steht, eine Verbindung der Formel

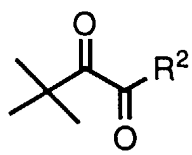 III oder V 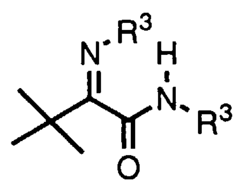

oder ein Gemisch davon,

worin $R^2$ für $NH_2$, nieder-Alkyl-NH, Phenyl-niederalkyl-NH oder nieder-Alkoxy und $R^3$ für nieder-Alkyl stehen, mit einem Amin der Formel

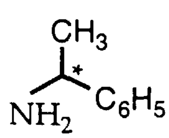 VI

umsetzt.

Die Reaktion a) wird in einem Lösungsmittel, wie Diethylether, bei einer Temperatur zwischen 0°C und Raumtemperatur durchgeführt.

Die Reaktion b) wird in einem apolaren Lösungsmittel, wie Benzol, Toluol oder Xylol, bei einer Temperatur bis zu Rückflusstemperatur des Reaktionsgemisches durchgeführt, wobei man vorzugsweise das entstehende Wasser sowie den gegebenenfalls entstehenden nieder-Alkanol oder ein gegebenenfalls entstehendes nieder-Alkyl-amin azeotrop abdestilliert.

Die Ester III, worin $R^2$ nieder-Alkoxy ist, erhält man

a) durch Veresterung der Trimethylbrenztraubensäure der Formel

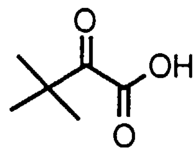

3

zum Beispiel in Gegenwart einer starken Säure, wie Salzsäure, in einem apolaren Lösungsmittel, wie Toluol, mittels dem entsprechenden nieder-Alkanol ($R^2$-H) bei einer Temperatur bis zur Rückflusstemperatur des Reaktionsgemisches, oder

b) durch Grignard-Reaktion zwischen dem entsprechenden Oxalsäurediester $(C(O)-R^2)_2$ in einem Lösungsmittel, wie Diethylether, mit einer Lösung von tert-Butylmagnesiumchlorid im gleichen Lösungsmittel, unter Abkühlung, z.B. auf -10°C.

Durch Reaktion eines wie oben beschrieben hergestellten Esters III mit einem nieder-Alkylamin oder Phenyl-niederalkylamin in einem Lösungsmittel, wie Ethanol, entsteht ein Ketoamid III, worin $R^2$ für $NH_2$, nieder-Alkyl-NH oder Phenyl-niederalkyl steht, eventuell im Gemisch mit einer Verbindung V.

Ein Ketoamid III bzw. dessen Gemisch mit V kann in seine Bestandteile aufgetrennt oder vorzugsweise durch Reaktion mit einem Amin der obigen Formel VI in das entsprechende α-Iminocarboxamid I, worin R nieder-alkylamino (NH-$R^3$) ist, umgesetzt werden. Durch Reaktion eines Esters III mit einem Amin VI entsteht das entsprechende α-Iminocarboxamid I, worin R für $R^0$ steht.

Die nachstehenden Beispiele a) bis n) bzw. 1 bis 12 veranschaulichen die Herstellung der Verbindungen I bzw. deren Hydrierung zu den Verbindungen II.

a) Zu 250 g einer wässrigen Lösung von Trimethylbrenztraubensäure (1,108 mol) und 62,5 ml konz. Salzsäure (0,38 mol) werden 125 ml Toluol zugesetzt. Das Reaktionsgemisch wird 2 Stunden unter Rückfluss am Wasserabscheider erhitzt. Das Destillat wird mit Toluol extrahiert und die Toluolphasen dem Reaktionsgemisch zugefügt. 100 ml Ethanol werden dem Reaktionsgemisch zugegeben und dieses 21 Stunden unter Rückfluss am Wasserabscheider erhitzt. Das wässrige Destillat wird mit Toluol extrahiert und die Toluolphasen dem Reaktionsgemisch zugefügt. Dann wird eine gesättigte Natriumbicarbonatlösung zugegeben und das Reaktionsgemisch 30 Minuten gerührt. Nach Trennung der Phasen wird die wässrige Phase mit Toluol extrahiert. Die vereinigten organischen Phasen werden mit einer Kochsalzlösung gewaschen, über Natriumsulfat getrocknet und filtriert. Das Natriumsulfat wird mit Toluol gewaschen. Die erhaltene Lösung enthält reinen Trimethylbrenztraubensäureethylester.

b) Dieser Lösung werden 300 ml einer Lösung von 33% Methylamin in Ethanol (2,409 mol) zugesetzt. Das Reaktionsgemisch wird 7 Stunden gerührt und dann eingeengt. Das Produkt (168,3 g) enthält 35% 3,3,N-Trimethyl-2-oxo-butyramid und 64% 3,3,N-Trimethyl-2-methylimino-butyramid.

c) Einer Lösung des Produkts von b) in 125 ml Toluol werden 147,5 g (S)-1-Phenylethylamin (1,217 mol) zugesetzt. Das Reaktionsgemisch wird 6 Stunden am Wasserabscheider unter Rückfluss gekocht, dann eingedampft und getrocknet. Man erhält 295,3 g (S)-3,3,N-Trimethyl-2-(1-phenylethylimino)-butyramid.

Zur Reinigung wird eine Lösung des Produkts von c) in 900 ml Toluol über basisches Aluminiumoxid filtriert. Das Filtrat wird eingedampft, der Rückstand bei 0°C aus Hexan kristallisiert und mit Hexan gewaschen. Man erhält 210,8 g reines (S)-3,3,N-Trimethyl-2-(1-phenylethylimino)-butyramid, Smp. 121,2-121,4°C, $[\alpha]_D^{20}$ = -75° (c = 10, $CHCl_3$).

d) Bei Verwendung von (R)-1-Phenylethylamin an Stelle des unter c) verwendeten (S)-1-Phenylethylamins erhält man das (R)-3,3,N-Trimethyl-2-(1-phenylethylimino)-butyramid, Smp. 119,5-120°C, $[\alpha]_D^{20}$ = +75° (c = 1,0, $CHCl_3$).

e) Eine Lösung von 12 g (82 mmol) Diethyloxalat in 100 ml Diethylether wird auf -18°C abgekühlt. Eine Lösung von 40 ml (80 mmol) 2M tert-Butylmagnesiumchlorid in Diethylether wird innert 2 Stunden so hinzugefügt, dass die Temperatur unterhalb -10°C bleibt. Nach einer Stunde Rühren bei -10°C wird das Reaktionsgemisch mit 40 ml einer gesättigten Ammoniumchloridlösung bei 0°C hydrolysiert und eine Stunde bei Raumtemperatur gerührt. Nach Aufarbeiten erhält man 220 ml einer Trimethyl-brenztraubensäureethylester enthaltenden Etherlösung. Gemäss gaschromatographischer Analyse ist dieser Ester 75% rein.

f) Dieser Lösung werden bei Raumtemperatur 20 ml (160 mmol) einer 33%igen Lösung von Methylamin in Ethanol zugesetzt. Nach 18 Stunden Rühren wird zu 9,1 g eines 69% 3,3,N-Trimethyl-2-oxo-butyramid und 13% 3,3,N-Trimethyl-2-methylimino-butyramid enthaltenden Oels eingedampft.

g) Dieses Oel wird in 30 ml Toluol gelöst und mit 12,1 g (100 mmol) (S)-1-Phenylethylamin versetzt. Das Reaktionsgemisch wird 46 Stunden am Wasserabscheider unter Rückfluss gekocht. Nach Eindampfen und Kristallisieren aus Hexan erhält man 8,84 g (45% basierend auf tert-Butyl-MgCl) (S)-3,3,N-Trimethyl-2-(1-phenylethylimino)-butyramid, Smp. 118,6-118,8°C, $[\alpha]_D^{20}$ = -74,4 (c = 0,9, $CHCl_3$).

h) Eine Lösung aus 15 g (95 mmol) Trimethyl-brenztraubensäureethylester und 50 g (413 mmol) (S)-1-Phenylethylamin in 50 ml Xylol wird am Wasserabscheider 18 Stunden unter Rückfluss gekocht. Anschliessend wird das Lösungsmittel abdestilliert und überschüssiges Amin im Vakum bei 120°C entfernt. Nach Kristallisation des Destillationsrückstandes aus 125 ml Hexan erhält man 14,3 g (45%) (S)-3,3-Dimethyl-N-[(S)-1-phenylethyl]-2-(1-phenylethylimino)-butyramid vom Schmelzpunkt 96-97°C. $[\alpha]_D^{20}$ = -58,9 (c = 1,0, $CHCl_3$).

i) Es wird wie unter h) beschrieben unter Verwendung von 15 g Trimethyl-brenztraubensäureethylester und 50 g (R)-1-Phenylethylamin verfahren. Man isoliert 14,9 g (47%) (R)-3,3-Dimethyl-N-[(R)-1-phenylethyl]-2-(1-phenylethylimino)-butyramid vom Schmelzpunkt 95-96°C. $[\alpha]_D^{20}$ = +59,6 (c = 1,0, $CHCl_3$).

j) Unter Ausschluss von Feuchtigkeit werden 8,9 g (47 mmol) (S)-(2,2-Dimethylpropyliden)-(1-phenylethyl)-amin (J.L. Fauchere, C. Petermann, Helv. Chim. Acta 1980, 63, 824) in 10 ml Diethylether gelöst. Zu dieser Lösung tropft man unter Eiskühlung 8,1 g (50 mmol) Trimethylbrenztraubensäure in 10 ml Diethylether. Anschliessend wird noch 2 Stunden bei 0°C und 6 Stunden bei Raumtemperatur gerührt. Während 2 Tagen Stehen bilden sich farblose Kristalle, die in 50 ml Diethylether suspendiert, filtriert und zweimal mit je 5 ml Diethylether gewaschen werden. Nach Trocknen im Vakuum erhält man 8,9 g (81%) (S)-3,3-Dimethyl-2-(1-phenylethylimino)-buttersäure (inneres Salz) vom Schmelzpunkt 135-136°C. $[\alpha]_D^{20}$ = -14,4 (c = 0,16, $CH_2Cl_2$).

k) (R)-3,3-Dimethyl-2-(1-phenylethylimino)-buttersäure (inneres Salz) lässt sich in analoger Weise aus (R)-(2,2-Dimethylpropyliden)-(1-phenylethyl)-amin und Trimethylbrenztraubensäure herstellen.

l) Eine Lösung aus 30,0 g (162 mmol) N-tert-butyl-3,3-dimethyl-2-oxobutyramid (D. Seyferth, R.C. Hui, Tetrahedron Lett. 1984, 25, 5251) und 50 g (413 mmol) (S)-1-Phenylethylamin in 150 ml Toluol wird am Wasserabscheider 20 Stunden unter Rückfluss gekocht. Anschliessend wird das Lösungsmittel und überschüssiges Amin im Vakuum entfernt. Der Rückstand wird mit 200 ml Hexan bei 0°C versetzt und gerührt. Nach Filtration der Suspension und Einengen im Vakuum wird das verbleibende Oel destilliert. Man erhält 37,2 g (80%) (S)-N-tert-Butyl-3,3-dimethyl-2-(1-phenylethylimino)-butyramid vom Siedepunkt 140°C bei 0,3 mbar. $[\alpha]_D^{20}$ = -40,7 (c = 1,0, $CHCl_3$).

m) Es wurde wie unter 1) beschrieben unter Verwendung von 7,5 g N-tert-Butyl-3,3-dimethyl-2-oxo-butyramid und 15 g (R)-1-Phenylethylamin verfahren. Man isoliert 6,8 g (58%) (R)-N-tert-Butyl-3,3-dimethyl-2-(1-phenylethylimino)-butyramid vom Siedepunkt 120° bei 0,2 mbar. $[\alpha]_D^{20}$ = +40,9 (c = 1,0, $CHCl_3$).

n) Eine Lösung aus 35,0 g (271 mmol) 3,3-Dimethyl-2-oxo-butyramid und 75,0 g (619 mmol) (S)-1-Phenylethylamin in 250 ml Toluol wird am Wasserabscheider 92 Stunden unter Rückfluss gekocht. Anschliessend wird das Lösungsmittel und überschüssiges Amin im Vakuum entfernt. Das verbleibende Oel wird aus 300 ml Hexan kristallisiert. Man erhält 51 g Rohmaterial vom Schmelzpunkt 96-97°C. Nach Umkristallisation aus 400 ml Hexan erhält man 42,7 g (68%) analysenreines (S)-3,3-Dimethyl-2-(1-phenylethylimino)-butyramid vom Schmelzpunkt 100-101°C. $[\alpha]_D^{20}$ = -58,5 (c = 1,0, $CHCl_3$).

o) Es wird wie unter n) beschrieben unter Verwendung von 5,0 g 3,3-Dimethyl-2-oxo-butyramid und 10,0 g (R)-1-Phenylethylamin verfahren. Nach einmaliger Kristallisation aus Hexan isoliert man 6,0 g (67%) (R)-3,3-Dimethyl-2-(1-phenylethylimino)-butyramid vom Schmelzpunkt 100-101°C. $[\alpha]_D^{20}$ = +61,4 (c = 0,8, $CHCl_3$).

Beispiel 1

In einem 2 1-Autoklav wird eine Lösung von 100,0 g (406 mmol) (S)-3,3,N-Trimethyl-2-(1-phenylethylimino)-butyramid in 775 ml Methanol in Gegenwart von 5 g 5-proz. Pd/C bei 40°C und bei einem Druck von 1 bar während 7 Stunden hydriert. Zur Vervollständigung der Hydrogenolyse wird während 6 Stunden bei 70°C und unter einem Druck von 2 bar nachhydriert. Nach dem Abkühlen wird der Katalysator abfiltriert und das Filtrat zur Trockene eingedampft: 58,3 g (99,6%) weisse Kristalle, die 99,6% N-Methyl-tert-leucinamid enthalten, e.e. 97,2%. Umkristallisation aus 425 ml Cyclohexan liefert 55,6 g (96,2%) (S)-N-Methyl-tert-leucinamid als weisse Kristalle, Smp. 90-93°C, $[\alpha]_D^{20}$ = -40,8 (c = 1, $CHCl_3$), 99,3% e.e.

Zur Bestimmung des e.e.-Wertes wird das Produkt mit (-)-Camphansäurechlorid derivatisiert und das Diastereomerengemisch mit Gaschromatographie analysiert.

Beispiel 2

In einem 380 ml-Autoklav wird eine Lösung von 3,0 g (12,18 mmol) (S)-3,3,N-Trimethyl-2-(1-phenylethylimino)-butyramid in 50 ml Methanol in Gegenwart von 0,5 g 5-proz. Pd/C bei 80°C und 2,0 bar Initialdruck während 5 Stunden hydriert. Dabei fällt der Druck auf 1,5 bar ab. Nach dem Abkühlen wird der Katalysator abfiltriert und das Filtrat zur Trockene eingedampft: 1,75 g (99,6%) weisse Kristalle, die 97,0% (S)-N-Methyl-tert-leucinamid enthielten, 93,7% e.e.

Beispiel 3

In einem Niederdruckhydrierapparat wird eine Lösung von 3,0 g (12,18 mmol) (R)-3,3,N-Trimethyl-2-(1-phenylethylimino)-butyramid in 50 ml Methanol in Gegenwart von 0,5 g 5-proz. Pd/C bei 40°C und Normaldruck während 21 Stunden hydriert. Nach dem Abkühlen wird der Katalysator abfiltriert und das Filtrat zur Trockene eingedampft: 1,75 g (99,6%) weisse Kristalle, die 99,5% (R)-N-Methyl-tert-leucinamid enthalten, 98,6% e.e.

Beispiel 4

In einem 380 ml-Autoklav wurde eine Lösung von 24,6 g (100 mmol) (S)-3,3,N-Trimethyl-2-(1-phenylethylimino)-butyramid in 180 ml Methanol und 10 ml Triethylamin in Gegenwart von 2,5 g 5-proz. Pd/C bei 20°C und einem Druck von 5 bar während 47 Stunden hydriert. Nach dem Entlasten wird der Katalysator abfiltriert und das Filtrat zur Trockene eingedampft. Man erhält 22,5 g (90,6%) weisse Kristalle, die 85,0% (S)-3,3,N-Trimethyl-2-((S)-1-phenylethylamino)-butyramid und 7,7% (R)-3,3,N-Trimethyl-2-((S)-1-phenylethylamino)-butyramid enthalten.

Zweimalige Umkristallisation aus 160 ml und 100 ml Diisopropylether liefert 11,5 g (51,3%) (S)-3,3,N-Trimethyl-2-[(S)-1-phenylethylamino]-butyramid als weisse Kristalle, Smp. 159-160°C, $[\alpha]_D^{20}$ = -107,9 (c = 1, $CHCl_3$).

Beispiel 5

In einem 380 ml-Autoklav wird eine Lösung von 12,4 g (50 mmol) (S)-3,3,N-Trimethyl-2-[(S)-1-phenylethylamino]-butyramid in 206 ml Methanol in Gegenwart von 1,2 g 5-proz. Pd/C bei 50°C und unter einem Druck von 4 bar während 6 Stunden hydriert. Nach dem Entlasten wird der Katalysator abfiltriert und das Filtrat zur Trockene eingedampft. Man erhält 7,1 g (98,5%) (S)-N-Methyl-tert-leucinamid als weisse Kristalle, 99,2% e.e.

Beispiel 6

In einem 380 ml-Autoklav wird eine Lösung von 24,6 g (100 mmol) (S)-3,3,N-Trimethyl-2-(1-phenylethylimino)-butyramid in 190 ml Methanol in Gegenwart von 2,5 g 5-proz. Pt/C bei 20°C und einem konstanten Druck von 5 bar während 23 Stunden hydriert. Nach dem Entlasten wird der Katalysator abfiltriert und das Filtrat zur Trockene eingedampft. Man erhält 23,2 g (93,3%) weisse Kristalle, die 85,2% (R)-3,3,N-Trimethyl-2-[(S)-1-phenylethylamino]-butyramid und 12,7% (S)-3,3,N-Trimethyl-2-[(S)-1-phenylethylamino]-butyramid enthalten.

Zweimalige Umkristallisation aus 90 ml und 85 ml Diisopropylether liefert 11,8 g (50,9%) (R)-3,3,N-Trimethyl-2-[(S)-1-phenylethylamino]-butyramid als weisse Kristalle, Smp. 107-108°C, $[\alpha]_D^{20}$ = +20,4 (c = 1, $CHCl_3$).

Beispiel 7

In einem Niederdruckhydrierapparat wird eine Lösung von 20,0 g (69,3 mmol) (S)-N-tert-Butyl-3,3-dimethyl-2-(1-phenylethylimino)-butyramid in 155 ml Methanol in Gegenwart von 12,5 g 5-proz. Pd/C bei 40°C und Normaldruck während 51 Stunden hydriert. Nach dem Abkühlen wird der Katalysator abfiltriert und das Filtrat zur Trockene eingedampft: 11,9 g (92,2%) weisse Kristalle, die 94,4% (S)-N-tert-Butyl-tert-leucinamid enthalten, 92,5% e.e.

Umkristallisation aus 50 ml Hexan liefert 7,8 g (66,0%) (S)-N-tert-Butyl-tert-leucinamid als weisse Kristalle, Smp. 78-80°C, $[\alpha]_D^{20}$= -32,2 (c = 1, $CHCl_3$), >98% e.e.

Beispiel 8

In einem Niederdruckhydrierapparat wird eine Lösung von 12,0 g (51,7 mmol) (S)-3,3-Dimethyl-2-(1-phenylethylimino)-butyramid in 200 ml Methanol in Gegenwart von 2,0 g 5-proz. Pd/C bei 40°C und Normaldruck während 6,5 Stunden hydriert. Nach dem Abkühlen wird der Katalysator abfiltriert und das Filtrat zur Trockene eingedampft: 6,7 g (99,5%) weisse Kristalle, die 90,8% (S)-tert-Leucinamid enthalten, 97,0% e.e. Umkristallisation aus 40 ml Cyclohexan liefert 5,3 g (78,5%) (S)-tert-Leucinamid als weisse Kristalle, Smp. 104-105°C, $[\alpha]_D^{20}$= -23,2 (c = 1, $CHCl_3$), 96,9% e.e.

Beispiel 9

In einem Niederdruckhydrierapparat wird eine Lösung von 3,0 g (8,9 mmol) (S)-3,3-Dimethyl-N-[(S)-1-phenylethyl]-2-(1-phenylethylimino)-butyramid in 200 ml Methanol in Gegenwart von 2,0 g 5-proz. Pd/C bei 40°C und Normaldruck während 6,5 Stunden hydriert. Nach dem Abkühlen wird der Katalysator abfiltriert und das Filtrat zur Trockene eingedampft: 6,7 g (99,5%) weisse Kristalle, die 90,8% (S)-N-[(S)-1-Phenylethyl]-tert-leucinamid enthalten, Smp. 57-60°C, $[\alpha]_D^{20}$ = -103,1 (c = 1, $CHCl_3$), 97,0% e.e.

Beispiel 10

In einem Niederdruckhydrierapparat wird eine Lösung von 2,0 g (8,6 mmol) (S)-3,3-Dimethyl-2-(1-phenylethylimino)-buttersäure (inneres Salz) in 60 ml Methanol in Gegenwart von 2,6 g (43 mmol) Essigsäure und 1,0 g 5-proz. Pd/C bei 40°C unter Normaldruck während 27 Stunden hydriert. Nach dem Abdühlen wird der Katalysator abfiltriert und das Filtrat zur Trockene eingedampft: 1,64 g weisser Schaum, 74,6 % e.e. Digerieren in 15 ml Ethanol liefert 0,85 g (74,5%) weisse Kristalle, die 99,5% (S)-tert-Leucin enthalten, 74,2% e.e.

Beispiel 11

In einem Niederdruckhydrierapparat wird eine Lösung von 2,0 g (8,6 mmol) (S)-3,3-Dimethyl-2-(1-phenylethylimino)-buttersäure (inneres Salz) in 60 ml Methanol in Gegenwart von 1,3 g (8,6 mmol) D-(-)-Weinsäure und 1,0 g 5-proz. Pd/C bei 40°C und Normaldruck während 24 Stunden hydriert. Nach dem Abkühlen wird der Katalysator abfiltriert und das Filtrat zur Trockene eingedampft: 2,55 g weisser Schaum, 84,1% e.e.

Digerieren in 15 ml Ethanol liefert 0,86 g (75,4%) weisse Kristalle, die 37,9% Weinsäure und 61,7% (S)-tert-Leucin enthalten, 100% e.e.

Beispiel 12

Eine Lösung von 2,0 g (S)-N-Methyl-tert-leucinamid in 100 ml einer 6N Lösung von $HCl/CH_3COOH$ (1:1) wird 14 Tage unter Rückfluss erhitzt. Dann wird eingedampft, der Rückstand über AMBERLITE® chromatographiert. Eindampfen und Trocknen ergeben 750 mg (41%) (S)-tert-Leucin, $[\alpha]_D$,$^{20}$ = +6,2 (c = 1,62, 5N HCl), 97% e.e.

Beispiel 13

Eine Lösung von 1,0 g (S)-N-tert-Butyl-tert-leucinamid in 10 ml 6N HCl wird 2 Tage bei 80°C erhitzt. Die Lösung wird zur Trockene eingedampft. Der Rückstand wird in Wasser gelöst und über AMBERLITE® chromatographiert. Man erhält 550 mg (78%) (S)-tert-Leucin, 100% e.e.

Beispiel 14

In zu Beispiel 13 analoger Weise wird 1 g (S)-tert-Leucinamid zu 250 mg (S)-tert-Leucin hydrolysiert, $[\alpha]_D$,$^{20}$ = +6,5 (c = 1,33, 5N HCl), 98% e.e.

**Patentansprüche**

1. Optisch aktive α-Iminocarbonsäurederivate der Formel

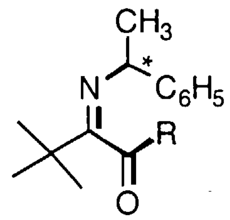

I

worin
R für OH, $NH_2$, ($C_1$-$C_6$)-Alkyl-NH oder Phenyl- ($C_1$-$C_6$) alkyl-NH steht.

2. α-Iminocarbonsäurederivate gemäss Anspruch 1, worin R

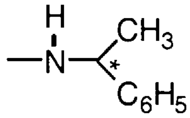 ($R^0$)

ist.

**3.** α-Iminocarbonsäurederivate gemäss Anspruch 1 oder 2, ausgewählt aus der Gruppe von

(S)-3,3,N-Trimethyl-2-(1-phenylethylimino)-butyramid,
(R)-3,3,N-Trimethyl-2-(1-phenylethylimino)-butyramid,
(S)-3,3-Dimethyl-N-[(S)-1-phenylethyl]-2-(1-phenylethylimino)-butyramid,
(S)-3,3-Dimethyl-2-(1-phenylethylimino)-buttersäure,
(S)-N-tert-Butyl-3,3-dimethyl-2-(1-phenylethylimino)-butyramid und
(S)-3,3-Dimethyl-2-(1-phenylethylimino)-butyramid, sowie
(R)-3,3-Dimethyl-N-[(R)-1-phenylethyl]-2-(1-phenylethylimino)-butyramid,
(R)-3,3-Dimethyl-2-(1-phenylethylimino)-buttersäure,
(R)-N-tert-Butyl-3,3-dimethyl-2-(1-phenylethylimino)-butyramid und
(R)-3,3-Dimethyl-2-(1-phenylethylimino)-butyramid.

**4.** Verfahren zur Herstellung der α-Aminocarbonsäurederivate der Formel

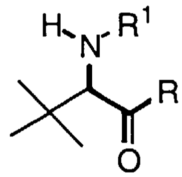 II

worin $R^1$ für H oder eine Gruppe der Formel

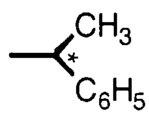 ($R^{10}$)

steht und R die gleiche Bedeutung wie in Anspruch 1 hat,
dadurch gekennzeichnet, dass man eine Verbindung der Formel I nach Anspruch 1 katalytisch hydriert, insbesondere unter Verwendung eines Platin- oder vorzugsweise Palladiumkatalysators.

**5.** Verfahren zur Herstellung der α-Iminocarbonsäurederivate der Formel I nach Anspruch 1, dadurch gekennzeichnet, dass man

a) zur Herstellung der α-Iminocarbonsäure der Formel I, worin R für OH steht, die Trimethylbrenztraubensäure

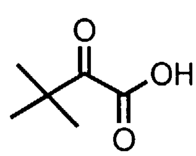

mit einer Verbindung der Formel

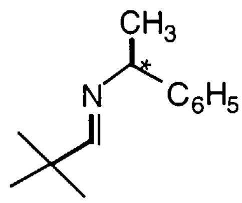 IV

umsetzt,

b) zur Herstellung eines α-Iminocarboxamids der Formel I, worin R für $NH_2$, $(C_1-C_6)$-Alkyl-NH oder Phenyl-$(C_1-C_6)$ alkyl-NH steht, eine Verbindung der Formel

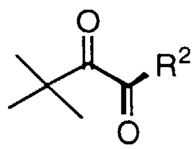 III oder V 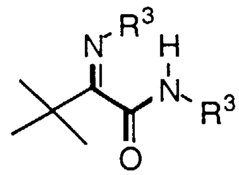

oder ein Gemisch davon, worin $R^2$ für $NH_2$, $(C_1-C_6)$-Alkyl-NH, Phenyl- $(C_1-C_6)$ alkyl-NH oder $(C_1-C_6)$-Alkoxy und $R^3$ für $(C_1-C_6)$-Alkyl stehen,

mit einem Amin der Formel

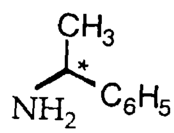 VI

umsetzt.

**Claims**

**1.** Optically active α-iminocarboxylic acid derivatives of the formula

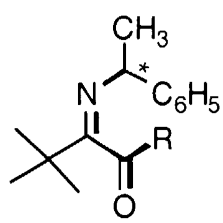 I

wherein
R stands for OH, $NH_2$, $(C_1-C_6)$-alkyl-NH or phenyl-$(C_1-C_6)$-alkyl-NH.

**2.** α-Iminocarboxylic acid derivatives according to claim 1, wherein R is

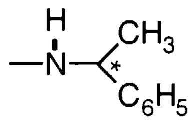

($R^0$).

3. α-Iminocarboxylic acid derivatives according to claim 1 or claim 2, selected from the group of

(S)-3,3,N-Trimethyl-2-(1-phenylethylimino)-butyramide,
(R)-3,3,N-trimethyl-2-(1-phenylethylimino)-butyramide,
(S)-3,3-dimethyl-N-[(S)-1-phenylethyl]-2-(1-phenylethylimino)-butyramide,
(S)-3,3-dimethyl-2-(1-phenylethylimino)-butyric acid,
(S)-N-tert-butyl-3,3-dimethyl-2-(1-phenylethylimino)-butyramide and
(S)-3,3-dimethyl-2-(1-phenylethylimino)-butyramide, as well as
(R)-3,3-dimethyl-N-[(R)-1-phenylethyl]-2-(1-phenylethylimino)-butyramide,
(R)-3,3-dimethyl-2-(1-phenylethylimino)-butyric acid,
(R)-N-tert-butyl-3,3-dimethyl-2-(1-phenylethylimino)-butyramide and
(R)-3,3-dimethyl-2-(1-phenylethylimino)-butyramide.

4. A process for the manufacture of the α-aminocarboxylic acid derivatives of the formula

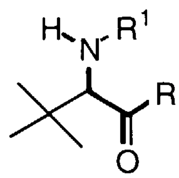

II

wherein $R^1$ stands for H or a group of the formula

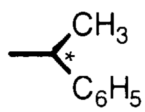

($R^{10}$)

and R has the same significance as in claim 1, which process comprises catalytically hydrogenating a compound of formula I according to claim 1, especially using a platinum catalyst or preferably a palladium catalyst.

5. A process for the manufacture of the α-iminocarboxylic acid derivatives of formula I according to claim 1, which process comprises

a) for the manufacture of the α-iminocarboxylic acid of formula I in which R stands for OH, reacting trimethylpyruvic acid

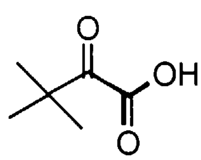

3

with a compound of the formula

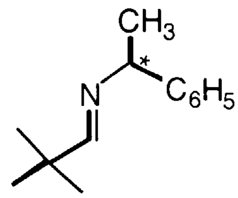 IV,

b) for the manufacture of an α-iminocarboxamide of formula I in which R stands for $NH_2$, $(C_1\text{-}C_6)$-alkyl-NH or phenyl-$(C_1\text{-}C_6)$-alkyl-NH, reacting a compound of the formula

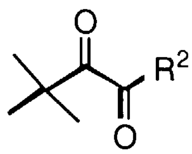 III or V 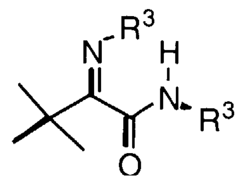

or a mixture thereof, wherein $R^2$ stands for $NH_2$, $(C_1\text{-}C_6)$-alkyl-NH, phenyl-$(C_1\text{-}C_6)$-alkyl-NH or lower-$(C_1\text{-}C_6)$ and $R^3$ stands for $(C_1\text{-}C_6)$-alkyl,

with an amine of the formula

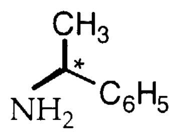 VI.

**Revendications**

1. Dérivés d'acide α-iminocarboxylique optiquement actifs de formule

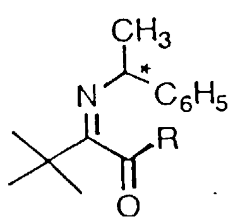 I

dans laquelle
R représente un groupe OH, $NH_2$, alkyl$(C_1\text{-}C_6)$-NH ou phényl-alkyl$(C_1\text{-}C_6)$-NH.

2. Dérivés d'acide α-iminocarboxylique selon la revendication 1, dans lesquels R est

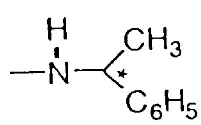 $(R^0)$

3. Dérivés d'acide α-iminocarboxylique selon la revendication 1 ou 2, choisis dans l'ensemble constitué par le (S)-3,3,N-triméthyl-2-(1-phényléthylimino)butyramide,

le (R)-3,3,N-triméthyl-2-(1-phényléthylimino)butyramide,
le (S)-3,3-diméthyl-N-[(S)-1-phényléthyl]-2-(1-phényléthylimino)-butyramide,
l'acide (S)-3,3-diméthyl-2-(1-phényléthylimino)butyrique,
le (S)-N-tert-butyl-3,3-diméthyl-2-(1-phényléthylimino)butyramide et
le (S)-3,3-diméthyl-2-(1-phényléthylimino)butyramide, ainsi que
le (R)-3,3-diméthyl-N-[(R)-1-phényléthyl]-2-(1-phényléthylimino)-butyramide,
l'acide (R)-3,3- diméthyl 2-(1-phényléthylimino)butyrique,
le (R)-N-tert-butyl-3,3-diméthyl-2-(1-phényléthylimino)butyramide et
le (R)-3,3-diméthyl-2-(1-phényléthylimino)butyramide.

**4.** Procédé pour la préparation des drivés d'acide α-iminocarboxylique de formule

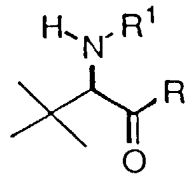

II

dans laquelle $R^1$ représente H ou un groupe de formule

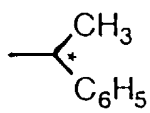

$(R^{10})$

et R a la même signification que dans la revendication 1, caractérisé en ce que l'on soumet à une hydrogénation catalytique un composé de formule I selon la revendication 1, en utilisant un catalyseur à base de platine ou de préférence de palladium.

**5.** Procédé pour la préparation des dérivés d'acide α-iminocarboxylique de formule I selon la revendication 1, caractérisé en ce que

a) pour la préparation de l'acide α-iminocarboxylique de formule I dans lequel R représente OH, on fait réagir l'acide triméthylpyruvique

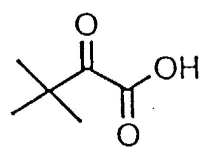

avec un composé de formule

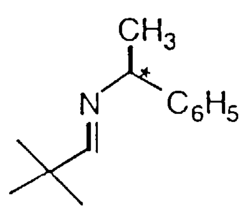

IV

b) pour la préparation d'un α-iminocarboxamide de formule I dans lequel R représente un groupe $NH_2$, alkyl $(C_1-C_6)$-NH ou phényl-alkyl$(C_1-C_6)$-NH, on fait réagir un composé de formule

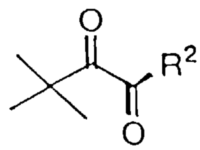

III ou V

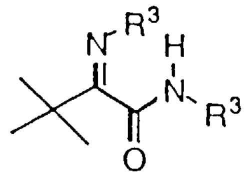

ou un mélange de tels composés

formules dans lesquelles $R^2$ représente un groupe $NH_2$, alkyl($C_1$-$C_6$)-NH, phényl-alkyl($C_1$-$C_6$)-NH ou alcoxy en $C_1$-$C_6$, et $R^3$ représente un groupe alkyle en $C_1$-$C_6$,

avec une amine de formule

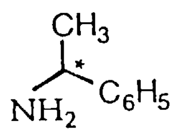

VI